# EUROPEAN PATENT SPECIFICATION

(11) **EP 1 253 932 B1**
(45) Date of publication and mention of the grant of the patent: **27.04.2005**
(21) Application number: 01905436.0
(22) Date of filing: 05.02.2001
(51) Int. Cl.: A61K 38/16, A61K 47/36, A61K 47/16, A61K 47/42, A61K 47/22

(54) **BOTULINUM TOXIN PHARMACEUTICAL COMPOSITIONS**
PHARMAZEUTISCHE ZUSAMMENSETZUNGEN MIT BOTULINUM TOXIN
COMPOSITIONS PHARMACEUTIQUES A BASE DE TOXINE BOTULIQUE

(30) Priority: 08.02.2000 US 500147
(43) Date of publication of application: 06.11.2002
(60) Divisional application: 03025104.5
(73) Proprietor: Allergan, Inc., Irvine, CA 92612 (US)
(72) Inventor: HUNT, Terrence, J., Anaheim Hills, CA 92807 (US)
(74) Representative: HOFFMANN - EITLE
(86) International application number: PCT/US2001/003641
(87) International publication number: WO 2001/058472

(56) References cited:
- WO-A-96/11699
- WO-A-97/35604
- GOODNOUGH MICHAEL C ET AL: "Stabilization of botulinum toxin type A during lyophilization." APPLIED AND ENVIRONMENTAL MICROBIOLOGY, vol. 58, no. 10, 1992, pages 3426-3428, XP001024270 ISSN: 0099-2240

## Description

### BACKGROUND

The present invention relates to pharmaceutical compositions. In particular, the present invention relates to pharmaceutical compositions containing a botulinum toxin and to methods for making the pharmaceutical compositions.

A pharmaceutical composition is a formulation containing one or more active ingredients as well as one or more excipients, carriers, stabilizers or bulking agents, which is suitable for administration to a human patient to achieve a desired diagnostic result or therapeutic effect.

For storage stability and convenience of handling, a pharmaceutical composition can be formulated as a lyophilized (i.e. freeze dried) or vacuum dried powder which can be reconstituted with saline or water prior to administration to a patient. Alternately, the pharmaceutical composition can be formulated as an aqueous solution. A pharmaceutical composition can contain a proteinaceous active ingredient. Unfortunately, proteins can be very difficult to stabilize, resulting in loss of protein and/or loss of protein activity during the formulation, reconstitution (if required) and during the storage prior to use of a protein containing pharmaceutical composition. Stability problems can occur because of protein denaturation, degradation, dimerization, and/or polymerization. Various excipients, such as albumin and gelatin have been used with differing degrees of success to try and stabilize a protein active ingredient present in a pharmaceutical composition. Additionally, cryoprotectants such as alcohols have been used to reduce protein denaturation under the freezing conditions of lyophilization.

### Albumin

Albumins are small, abundant plasma proteins. Human serum albumin has a molecular weight of about 69 kiloDaltons (kD) and has been used as a non-active ingredient in a pharmaceutical composition where it can serve as a bulk carrier and stabilizer of certain protein active ingredients present in a pharmaceutical composition.

The stabilization function of albumin in a pharmaceutical composition can be present both during the multistep formulation of the pharmaceutical composition and upon the later reconstitution of the formulated pharmaceutical composition. Thus, stability can be imparted by albumin to a proteinaceous active ingredient in a pharmaceutical composition by, for example, (1) reducing adhesion (commonly referred to as "stickiness") of the protein active ingredient to surfaces, such as the surfaces of laboratory glassware, vessels, to the vial in which the pharmaceutical composition is reconstituted and to the inside surface of a syringe used to inject the pharmaceutical composition. Adhesion of a protein active ingredient to surfaces can led to loss of active ingredient and to denaturation of the remaining retained protein active ingredient, both of which reduce the total activity of the active ingredient present in the pharmaceutical composition, and; (2) reducing denaturation of the active ingredient which can occur upon preparation of a low dilution solution of the active ingredient.

As well as being able to stabilize a protein active ingredient in a pharmaceutical composition, albumin also has the advantage of generally negligible immunogenicity when injected into a human patient. A compound with an appreciable immunogenicity can cause the production of antibodies against it which can lead to an anaphylactic reaction and/or to the development of drug resistance, with the disease or disorder to be treated thereby becoming potentially refractory to the pharmaceutical composition which has an immunogenic component.

Unfortunately, despite its known stabilizing effect, significant drawbacks exist to the use of albumin in a pharmaceutical composition. For example albumins are expensive and increasingly difficult to obtain. Furthermore, blood products such as albumin, when administered to a patient can subject the patient to a potential risk of receiving blood borne pathogens or infectious agents. Thus, it is known that the possibility exists that the presence of albumin in a pharmaceutical composition can result in inadvertent incorporation of infectious elements into the pharmaceutical composition. For example, it has been reported that use of albumin may transmit prions into a pharmaceutical composition. A prion is a proteinaceous infectious particle which is hypothesized to arise as an abnormal conformational isoform from the same nucleic acid sequence which makes the normal protein. It has been further hypothesized that infectivity resides in a "recruitment reaction" of the normal isoform protein to the prion protein isoform at a post translational level. Apparently the normal endogenous cellular protein is induced to misfold into a pathogenic prion conformation. Significantly, several lots of human serum albumin have been withdrawn from distribution upon a determination that a blood donor to a pool from which the albumin was prepared was diagnosed with Creutzfeldt-Jacob disease.

Creutzfeldt-Jacob disease (sometimes characterized as Alzheimer's disease on fast forward) is a rare neurodegenerative disorder of human transmissible spongiform encephalopathy where the transmissible agent is apparently an abnormal isoform of a prion protein. An individual with Creutzfeldt-Jacob disease can deteriorate from apparent perfect health to akinetic mutism within six months. Possible iatrogenic transmission of Creutzfeldt-Jacob disease by albumin transfusion has been reported and it has been speculated that sufficient protection against Creutzfeldt-Jacob disease transmission is not provided by the usual methods of albumin preparation which methods include disposal of blood cellular elements and heating to 60 degrees C. for 10 hours. Thus a potential risk may exist of acquiring a prion mediated disease, such Creutzfeldt-Jacob disease, from the administration of a pharmaceutical composition which contains human plasma protein concentrates, such as serum albumin.

Gelatin has been used in some protein active ingredient pharmaceutical compositions as an albumin substitute. Notably, gelatin is a animal derived protein and therefore carries the same risk of potential infectivity which may be possessed by albumin. Hence, it is desirable to find a substitute for albumin which is not a blood fraction, and preferably, the albumin substitute is not gelatin and is not derived from any animal source.

### Botulinum toxin

The anaerobic, gram positive bacterium *Clostridium botulinum* produces a potent polypeptide neurotoxin, botulinum toxin, which causes a neuroparalytic illness in humans and animals referred to as botulism. Clostridium botulinum and its spores are commonly found in soil and the bacterium can grow in improperly sterilized and sealed food containers of home based canneries, which are the cause of many of the cases of botulism. The effects of botulism typically appear 18 to 36 hours after eating the foodstuffs infected with a Clostridium botulinum culture or spores. The botulinum toxin can apparently pass unattenuated through the lining of the gut and attack peripheral motor neurons. Symptoms of botulinum toxin intoxication can progress from difficulty walking, swallowing, and speaking to paralysis of the respiratory muscles and death.

Botulinum toxin type A is the most lethal natural biological agent known to man. About 50 picograms of botulinum toxin (purified neurotoxin complex) type A is a LD₅₀ in mice. Interestingly, on a molar basis, botulinum toxin type A is 1.8 billion times more lethal than diphtheria, 600 million times more lethal than sodium cyanide, 30 million times more lethal than cobrotoxin and 12 million times more lethal than cholera. Singh, *Critical Aspects of Bacterial Protein Toxins,* pages 63-84 (chapter 4) of Natural Toxins II, edited by B.R. Singh et al., Plenum Press, New York (1976) (where the stated LD₅₀ of botulinum toxin type A of 0.3 ng equals 1 U is corrected for the fact that about 0.05 ng of BOTOX® equals 1 unit). One unit (U) of botuiinum toxin is defined as the LD₅₀ upon intraperitoneal injection into female Swiss Webster mice weighing 18-20 grams each. Seven immunologically distinct botulinum neurotoxins have been characterized, these being respectively botulinum neurotoxin serotypes A, B, C₁, D, E, F and G each of which is distinguished by neutralization with type-specific antibodies. The different serotypes of botulinum toxin vary in the animal species that they affect and in the severity and duration of the paralysis they evoke. For example, it has been determined that botulinum toxin type A is 500 times more potent, as measured by the rate of paralysis produced in the rat, than is botulinum toxin type B. Additionally, botulinum toxin type B has been determined to be non-toxic in primates at a dose of 480 U/kg which is about 12 times the primate LD₅₀ for botulinum toxin type A. The botulinum toxins apparently bind with high affinity to cholinergic motor neurons, are translocated into the neuron and block the presynaptic release of acetylcholine.

Botulinum toxins have been used in clinical settings for the treatment of neuromuscular disorders characterized by hyperactive skeletal muscles. Botulinum toxin type A was approved by the U.S. Food and Drug Administration in 1989 for the treatment of essential blepharospasm, strabismus and hemifacial spasm in patients over the age of twelve. Clinical effects of peripheral intramuscular botulinum toxin type A are usually seen within one week of injection. The typical duration of symptomatic relief (i.e. flaccid muscle paralysis) from a single intramuscular injection of botulinum toxin type A can be about three months.

Although all the botulinum toxins serotypes apparently inhibit release of the neurotransmitter acetylcholine at the neuromuscular junction, they do so by affecting different neurosecretory proteins and/or cleaving these proteins at different sites. Botulinum toxin A is a zinc endopeptidase which can specifically hydrolyze a peptide linkage of the intracellular, vesicle associated protein SNAP-25. Botulinum type E also cleaves the 25 kiloDalton (kD) synaptosomal associated protein (SNAP-25), but targets different amino acid sequences within this protein, as compared to botulinum toxin type A. Botulinum toxin types B, D, F and G act on vesicle-associated protein (VAMP, also called synaptobrevin), with each serotype cleaving the protein at a different site. Finally, botulinum toxin type C₁ has been shown to cleave both syntaxin and SNAP-25. These differences in mechanism of action may affect the relative potency and/or duration of action of the various botulinum toxin serotypes.

The molecular weight of the botulinum toxin protein molecule, for all seven of the known botulinum toxin serotypes, is about 150 kD. Interestingly, the botulinum toxins are released by Clostridial bacterium as complexes comprising the 150 kD botulinum toxin protein molecule along with associated non-toxin proteins. Thus, the botulinum toxin type A complex can be produced by Clostridial bacterium as 900 kD, 500 kD and 300 kD forms. Botulinum toxin types B and C₁ is apparently produced as only a 500 kD complex. Botulinum toxin type D is produced as both 300 kD and 500 kD complexes. Finally, botulinum toxin types E and F are produced as only approximately 300 kD complexes. The complexes (i.e. molecular weight greater than about 150 kD) are believed to contain a non-toxin hemaglutinin protein and a non-toxin and non-toxic nonhemaglutinin protein. These two non-toxin proteins (which along with the botulinum toxin molecule can comprise the relevant neurotoxin complex) may act to provide stability against denaturation to the botulinum toxin molecule and protection against digestive acids when toxin is ingested. Additionally, it is possible that the larger (greater than about 150 kD molecular weight) botulinum toxin complexes may result in a slower rate of diffusion of the botulinum toxin away from a site of intramuscular injection of a botulinum toxin complex. The toxin complexes can be dissociated into toxin protein and hemagglutinin proteins by treating the complex with red blood cells at pH 7.3. The toxin protein has a marked instability upon removal of the hemagglutinin protein.

All the botulinum toxin serotypes made by Clostridium botulinum bacteria as inactive single chain proteins which must be cleaved or nicked by proteases to become neuroactive. The bacterial strains that make botulinum toxin serotypes A and G possess endogenous proteases and serotypes A and G can therefore be recovered from bacterial cultures in predominantly their active form. In contrast, botulinum toxin serotypes C₁, D and E are synthesized by nonproteolytic strains and are therefore typically unactivated when recovered from culture. Serotypes B and F are produced by both proteolytic and nonproteolytic strains and therefore can be recovered in either the active or inactive form. However, even the proteolytic strains that produce, for example, the botulinum toxin type B serotype only cleave a portion of the toxin produced. The exact proportion of nicked to unnicked molecules depends on the length of incubation and the temperature of the culture. Therefore, a certain percentage of any preparation of, for example, the botulinum toxin type B toxin is likely to be inactive, possibly accounting for the known significantly lower potency of botulinum toxin type B as compared to botulinum toxin type A. The presence of inactive botulinum toxin molecules in a clinical preparation will contribute to the overall protein load of the preparation, which has been linked to increased antigenicity, without contributing to its clinical efficacy. Additionally, it is known that botulinum toxin type B has, upon intramuscular injection, a shorter duration of activity and is also less potent than botulinum toxin type A at the same dose level.

High quality crystalline botulinum toxin type A can be produced from the Hall A strain of Clostridium botulinum with characteristics of ≥3 X 10⁷ U/mg, an A₂₆₀/A₂₇₈ of less than 0.60 and a distinct pattern of banding on gel electrophoresis. The known Shantz process can be used to obtain crystalline botulinum toxin type A, as set forth in Shantz, E.J., et al, *Properties and use of Botulinum toxin and Other Microbial Neurotoxins in Medicine,* Microbiol Rev. 56: 80-99 (1992). Generally, the botulinum toxin type A complex can be isolated and purified from an anaerobic fermentation by cultivating *Clostridium botulinum* type A in a suitable medium. Raw toxin can be harvested by precipitation with sulfuric acid and concentrated by ultramicrofiltration. Purification can be carried out by dissolving the acid precipitate in calcium chloride. The toxin can then be precipitated with cold ethanol. The precipitate can dissolved in sodium phosphate buffer and centrifuged. Upon drying there can then be obtained approximately 900 kD crystalline botulinum toxin type A complex with a specific potency of 3 X 10⁷ LD₅₀ U/mg or greater. This known process can also be used, upon separation out of the non-toxin proteins, to obtain pure botulinum toxins, such as for example: purified botulinum toxin type A with an approximately 150 kD molecular weight with a specific potency of 1-2 X 10⁸ LD₅₀ U/mg or greater; purified botulinum toxin type B with an approximately 156 kD molecular weight with a specific potency of 1-2 X 10⁸ LD₅₀ U/mg or greater, and; purified botulinum toxin type F with an approximately 155 kD molecular weight with a specific potency of 1-2 X 10⁷ LD₅₀ U/mg or greater.

Already prepared and purified botulinum toxins and toxin complexes suitable for preparing pharmaceutical formulations can be obtained from List Biological Laboratories, Inc., Campbell, California; the Centre for Applied Microbiology and Research, Porton Down, U.K.; Wako (Osaka, Japan), as well as from Sigma Chemicals of St Louis, Missouri.

Pure botulinum toxin is so labile that it is has limited practical utility to prepare a pharmaceutical composition. Furthermore. the botulinum toxin complexes, such a the toxin type A complex are also extremely susceptible to denaturation due to surface denaturation, heat, and alkaline conditions. Inactivated toxin forms toxoid proteins which may be immunogenic. The resulting antibodies can render a patient refractory to toxin injection.

As with enzymes generally, the biological activities of the botulinum toxins (which are intracellular peptidases) is dependant, at least in part, upon their three dimensional conformation. Thus, botulinum toxin type A is detoxified by heat, various chemicals surface stretching and surface drying. Additionally, it is known that dilution of the toxin complex obtained by the known culturing, fermentation and purification to the much, much lower toxin concentrations used for pharmaceutical composition formulation results in rapid detoxification of the toxin unless a suitable stabilizing agent is present. Dilution of the toxin from milligram quantities to a solution containing nanograms per milliliter presents significant difficulties because of the rapid loss of specific toxicity upon such great dilution. Since the toxin may be used months or years after the toxin containing pharmaceutical composition is formulated, the toxin must be stabilized with a stabilizing agent. The only successful stabilizing agent for this purpose has been the animal derived proteins albumin and gelatin. And as indicated, the presence of animal derived proteins in the final formulation presents potential problems in that certain stable viruses, prions or other infectious or pathogenic compounds carried through from donors can contaminate the toxin.

Furthermore, any one of the harsh pH, temperature and concentration range conditions required to lyophilize (freeze-dry) or vacuum dry a botulinum toxin containing pharmaceutical composition into a toxin shipping and storage format (ready for use or reconstitution by a physician) can detoxify the toxin. Thus, animal derived or donor pool proteins such as gelatin and serum albumin have been used with some success to stabilize botulinum toxin.

A commercially available botulinum toxin containing pharmaceutical composition is sold under the trademark BOTOX® (available from Allergan, Inc., of Irvine, California). BOTOX® consists of a purified botulinum toxin type A complex, albumin and sodium chloride packaged in sterile, vacuum-dried form. The botulinum toxin type A is made from a culture of the Hall strain of Clostridium botulinum grown in a medium containing N-Z amine and yeast extract. The botulinum toxin type A complex is purified from the culture solution by a series of acid precipitations to a crystalline complex consisting of the active high molecular weight toxin protein and an associated hemagglutinin protein. The crystalline complex is re-dissolved in a solution containing saline and albumin and sterile filtered (0.2 microns) prior to vacuum-drying. BOTOX® can be reconstituted with sterile, non-preserved saline prior to intramuscular injection. Each vial of BOTOX® contains about 100 units (U) of Clostridium botulinum toxin type A complex, 0.5 milligrams of human serum albumin and 0.9 milligrams of sodium chloride in a sterile, vacuum-dried form without a preservative.

To reconstitute vacuum-dried BOTOX® sterile normal saline without a preservative; 0.9% Sodium Chloride Injection is used by drawing up the proper amount of diluent in the appropriate size syringe. Since BOTOX® is denatured by bubbling or similar violent agitation, the diluent is gently injected into the vial. BOTOX® should be administered within four hours after reconstitution. During this time period, reconstituted BOTOX® is stored in a refrigerator (2° to 8°C). Reconstituted BOTOX® is clear, colorless and free of particulate matter. The vacuum-dried product is stored in a freezer at or below - 5°C. BOTOX® is administered within four hours after the vial is removed from the freezer and reconstituted. During these four hours, reconstituted BOTOX® can be stored in a refrigerator (2° to 8°C).

It has been reported that a suitable alternative to albumin as a botulinum toxin stabilizer may be another protein or alternatively a low molecular weight (non-protein) compound. Carpender et al., I*nteractions of Stabilizing Additives with Proteins During Freeze-Thawing and Freeze-Drying,* International Symposium on Biological Product freeze-Drying and Formulation, 24-26 October 1990; Karger (1992), 225-239.

Many substances commonly used as carriers and bulking agents in pharmaceutical compositions have proven to be unsuitable as albumin replacements in the neurotoxin containing pharmaceutical composition. For example, the disaccharide cellobiose has been found to be unsuitable as a toxin stabilizer. Thus, it is known that the use of cellobiose as an excipient in conjunction with albumin and sodium chloride results in a much lower level of toxicity (10% recovery) after lyophilization of crystalline botulinum toxin type A with these excipients, as compared to the toxicity after lyophilization with only albumin (>75% to >90% recovery). Goodnough et al., *Stabilization of Botulinum Toxin Type A During Lyophilization,* App & Envir. Micro. 58 (10) 3426-3428 (1992).

Furthermore, saccharides, including polysaccharides, are in general poor candidates to serve as protein stabilizers. Thus, it is known that a pharmaceutical composition containing a protein active ingredient is inherently unstable if the protein formulation comprises a saccharide (such as glucose or a polymer of glucose) or carbohydrates because proteins and glucose are known to interact together and to undergo the well-described Maillard reaction, due, i.e. to the reducing nature of glucose and glucose polymers. Much work has been dedicated to mostly unsuccessful attempts at preventing this protein-saccharide reaction by, for example, reduction of moisture or use of non-reducing sugars. Significantly, the degradtive pathway of the Maillard reaction can result in a therapeutic insufficiency of the protein active ingredient. A pharmaceutical formulation comprising protein and a reducing saccharide, carbohydrate or sugar, such as a glucose polymer, is therefore inherently unstable and cannot be stored for a long period of time without significant loss of the active ingredient protein's desired biological activity.

Notably, one of the reasons human serum albumin can function effectively as a stabilizer of a protein active ingredient in a pharmaceutical composition is because, albumin, being a protein, does not undergo the Maillard reaction with the protein active ingredient in a pharmaceutical composition. Hence, one would expect to find and to look for a substitute for albumin amongst other proteins.

Finding an appropriate substitute for albumin as a stabilizer of the botulinum toxin present in a pharmaceutical composition is difficult and problematic because albumin is believed to function in a pharmaceutical composition as more than a mere bulking agent. Thus, albumin apparently can interact with botulinum toxin so as to increase the potency of the neurotoxin. For example, it is known that bovine serum albumin can act as more than a mere stabilizing excipient for botulinum toxin type A, since bovine serum albumin apparently also accelerates the rate of catalysis of synthetic peptide substrates, which substrates resemble the SNAP-25 intraneuronal substrate for botulinum toxin type A Schmidt, et al., *Endoproteinase Activity of Type A Botulinum Neurotoxin Substrate Requirements and Activation by* *Serum Albumin,* J. of Protein Chemistry, 16 (1), 19-26 (1997). Thus, albumin may have a potentiating effect, apparently by affecting rate kinetics, upon the intracellular proteolytic action of a botulinum toxin upon the toxin's substrate. This potentiating effect may be due to albumin which has accompanied the botulinum toxin upon endocytosis of the toxin into a target neuron or the potentiating effect may be due to the pre-existing presence cytoplasmic albumin within the neuron protein prior to endocytosis of the botulinum toxin.

The discovery of the presence of a kinetic rate stimulatory effect by bovine serum albumin upon the proteolytic activity of botulinum toxin type A renders the search for a suitable substitute for albumin in a botulinum toxin containing pharmaceutical formulation especially problematic. Thus, an albumin substitute with desirable toxin stabilization characteristics may have an unknown and possibly deleterious effect upon the rate of substrate catalysis by the toxin, since at least with regard to bovine serum albumin the two characteristics (toxin stabilization and toxin substrate catalysis potentiation) are apparently inherent to the same albumin excipient. This potentiating effect of albumin shows that albumin does not act as a mere excipient in the formulation and therefore renders the search for a suitable substitute for albumin more difficult.

Additionally there are many unique characteristics of botulinum toxin and its formulation into a suitable pharmaceutical composition which constrain and hinder and render the search for a replacement for the albumin used in current botulinum toxin containing pharmaceutical formulations very problematic. Examples of four of these unique characteristics follow. First, botulinum toxin is a relatively large protein for incorporation into a pharmaceutical formulation (the molecular weight of the botulinum toxin type A complex is 900 kD) and is therefore is inherently fragile and labile. The size of the toxin complex makes it much more friable and labile than smaller, less complex proteins, thereby compounding the formulation and handling difficulties if toxin stability is to be maintained. Hence, an albumin replacement must be able to interact with the toxin in a manner which does not denature, fragment or otherwise detoxify the toxin molecule or cause disassociation of the non-toxin proteins present in the toxin complex. Second, as the most lethal known biological product, exceptional safety, precision, and accuracy is called for at all steps of the formulation of a botulinum toxin containing pharmaceutical composition. Thus, a preferred potential albumin replacer should not itself be toxic or difficult to handle so as to not exacerbate the already extremely stringent botulinum toxin containing pharmaceutical composition formulation requirements.

Third, since botulinum toxin was the first microbial toxin to be approved for injection for the treatment of human disease, specific protocols had to be developed and approved for the culturing, bulk production, formulation into a pharmaceutical and use of botulinum toxin. Important considerations are toxin purity and dose for injection. The production by culturing and the purification must be carried out so that the toxin is not exposed to any substance that might contaminate the final product in even trace amounts and cause undue reactions in the patient. These restrictions require culturing in simplified medium without the use of animal meat products and purification by procedures not involving synthetic solvents or resins. Preparation of toxin using enzymes, various exchangers, such as those present in chromatography columns and synthetic solvents can introduce contaminants and are therefore excluded from preferred formulation steps. Furthermore, botulinum toxin type A is readily denatured at temperatures above 40 degrees C., loses toxicity when bubbles form at the air/liquid interface, and denatures in the presence of nitrogen or carbon dioxide.

Fourth, particular difficulties exist to stabilize botulinum toxin type A, because type A consists of a toxin molecule of about 150 kD in noncovalent association with nontoxin proteins weighing about 750 kD. The nontoxin proteins are believed to preserve or help stabilize the secondary and tertiary structures upon which toxicity is dependant. Procedures or protocols applicable to the stabilization of nonproteins or to relatively smaller proteins are not applicable to the problems inherent with stabilization of the botulinum toxin complexes, such as the 900 kD botulinum toxin type A complex. Thus while from pH 3.5 to 6.8 the type A toxin and non toxin proteins are bound together noncovalently, under slightly alkaline conditions (pH > 7.1) the very labile toxin is released from the toxin complex. Because of its lability, pure toxin has no or limited utility for medical administration.

In light of the unique nature of botulinum toxin and the requirements set forth above, the probability of finding a suitable albumin replacement for the albumin used in current botulinum toxin containing pharmaceutical compositions must realistically be seen to approach zero. Prior to the present invention, only the animal derived proteins albumin and gelatin had been known to have utility as suitable stabilizers of the botulinum toxin present in a pharmaceutical formulation. Thus, albumin, by itself or with one or additional substances such as sodium phosphate or sodium citrate, is known to permit high recovery of toxicity of botulinum toxin type A after lyophilization. Unfortunately, as already set forth, albumin, as a pooled blood product, can, at least potentially, carry infectious or disease causing elements when present in a pharmaceutical composition. Indeed, any animal product or protein such as gelatin can also potentially contain pyrogens or other substances that can cause adverse reactions upon injection into a patient.

Chinese patent application CN 1215084A discusses an albumin free botulinum toxin type A formulated with gelatin, an animal derived protein. This formulation therefore does not eliminate the risk of transmitting an animal protein derived or accompanying infectious element.

### Hydroxyethyl Starch

A polysaccharide can be made up of hundreds or even thousands of monosaccharide units held together by glycoside (ether) linkages. Two important polysaccharides are cellulose and starch. Cellulose is the chief structural material in plants, giving plants their rigidity and form. Starch makes up the reserve food supply of plants and is found mainly in various seeds and tubers.

Starch occurs as granules whose size and shape are characteristic of the plant from which the starch is obtained. In general about 80% of starch is a water insoluble fraction called amylopectin. Amylopectin is made up of chains of D-glucose (as glucopyranose) units, each unit being joined by an alpha glycoside linkage to C-4 of the next glucose unit. Like starch, cellulose is also made up of chains of D-glucose units, where each unit is joined by a glucoside linkage to the C-4 of the next unit. Unlike starch though, the glycoside linkages in cellulose are beta linkages. Treatment of cellulose with sulfuric acid and acetic anhydride yields the disaccharide cellobiose. As previously set forth, attempts to stabilize botulinum toxin using cellobiose have been unsuccessful.

A particular starch derivative which can be obtained by treating starch with pyridine and ethylene chlorohydrin, is 2-hydroxyethyl starch, also called hetastarch. U.S. patent 4,457,916 discloses a combination of a nonionic surfactant and hydroxyethyl starch to stabilize aqueous solutions of tumor necrosis factor (TNF). Additionally, a 6% aqueous solution of 2-hydroxyethyl starch (hetastarch) (available from Du Pont Pharma, Wilmington, Delaware under the trade name HESPAN®, 6% hetastarch in 0.9% sodium chloride injection) is known. Albumin is known to act as a plasma volume expander upon intravenous administration to a patient. HESPAN® has also been administrated to patients to achieve a plasma volume expansion effect and in that sense intravenous HESPAN® can be considered a replacement for intravenous albumin.

Hetastarch it is an artificial colloid derived from a waxy starch composed almost entirely of amylopectin. Hetastarch can be obtained by introducing hydroxyethyl ether groups onto glucose units of the starch, and the resultant material can then be hydrolyzed to yield a product with a molecular weight suitable for use as a plasma volume expander. Hetastarch is characterized by its molar substitution and also by its molecular weight. The molar substitution can be approximately 0.75, meaning that hetastarch is etherified to the extent that for every 100 glucose units of hetastarch there are, on average, approximately 75 hydroxyethyl substituent groups. The weight average molecular weight of hetastarch is approximately 670 kD with a range of 450 kD to 800 kD and with at least 80% of the polymer units falling within the range of 20 kD to 2,500 kD. Hydroxyethyl groups are attached by ether linkages primarily at C-2 of the glucose unit and to a lesser extent at C-3 and C-6. The polymer resembles glycogen, and the polymerized D-glucose units are joined primarily by α-1,4 linkages with occasional α-1,6 branching linkages. The degree of branching is approximately 1:20, meaning that there is an average of approximately one α-1,6 branch for every 20 glucose monomer units. Hetastarch is comprised of more than 90% amylopectin.

The plasma volume expansion produced by HESPAN® can approximate that obtained with albumin. Hetastarch molecules below 50 kD molecular weight are rapidly eliminated by renal excretion and a single dose of approximately 500 mL of HESPAN® (approximately 30 g) results in elimination in the urine of approximately 33% of the administered HESPAN® within about 24 hours. The hydroxyethyl group of hydroxyethyl starch is not cleaved *in vivo,* but remains intact and attached to glucose units when excreted. Significant quantities of glucose are not produced as hydroxyethylation prevents complete metabolism of the smaller hydroxyethyl starch polymers

Cellulose can likewise be converted to a hydroxyethyl cellulose. The average molecular weight of 2-hydroxyethyl cellulose (a 2-hydroxyethyl ether of cellulose) is about 90 kD. Unfortunately, hydroxyethyl cellulose, unlike hydroxyethyl starch, is highly reactive and therefore unsuited for use as a stabilizer of a protein active ingredient in a pharmaceutical formulation.

What is needed therefore is a suitable replacement for the animal derived protein or donor pool albumin stabilizer used in neurotoxin containing pharmaceutical compositions.

WO 96/11699 discloses pharmaceutical compositions comprising essentially pure botulinum toxin type A, a stabilizing protein, a polyhydroxy compound and water. The polyhydroxy compound is preferably selected from trehalose, maltotriose and related compounds (cf. also Goodnough et al. in Applied an Environmental Microbiology (1992) 3426).

WO 97/35604 discloses lyophilized pharmaceutical compositions containing botulinum toxin or botulinum neurotoxin and effective amounts of trehalose and methionine.

### SUMMARY

The present invention meets this need and provides a replacement for the albumin present in a pharmaceutical composition by a compound which can which can stabilize the botulinum toxin present in the pharmaceutical composition. The albumin replacement compound has the characteristic of low, and preferably negligible, immunogenicity when injected into a human patient. Additionally, the preferred albumin replacer has a rapid rate of clearance from the body after injection of the pharmaceutical composition.

### Definitions

As used herein, the words or terms set forth below have the following definitions.

The word "about" means that the item, parameter or term so qualified encompasses a range of plus or minus ten percent above and below the value of the stated item, parameter or term.

The phrase "amino acid" includes polyamino acids.

The word "polysaccharide" means a polymer of more than two saccharide molecule monomers, which monomers can be identical or different.

The phrase "pharmaceutical composition" means a formulation in which an active ingredient is a neurotoxin, such as a Clostridial neurotoxin. The word "formulation" means that there is at least one additional ingredient in the pharmaceutical composition besides the neurotoxin active ingredient. A pharmaceutical composition is therefore a formulation which is suitable for diagnostic or therapeutic administration (i.e. by intramuscular or subcutaneous injection) to a human patient. The pharmaceutical composition can be: in a lyophilized or vacuum dried condition; a solution formed after reconstitution of the lyophilized or vacuum dried pharmaceutical composition with saline or water, or; as a solution which does not require reconstitution. The neurotoxin active ingredient can be one of the botulinum toxin serotypes A, B, C₁, D, E, F or G or a tetanus toxin, all of which are made by Clostridial bacteria.

The phrase "therapeutic formulation" means a formulation can be used to treat and thereby to alleviate a disorder or a disease, such as a disorder or a disease characterized by hyperactivity (i.e. spasticity) of a peripheral muscle.

The words "stabilizing", "stabilizes" or "stabilization" mean that upon reconstitution with saline or water of a lyophilized, or vacuum dried botulinum toxin containing pharmaceutical composition which has been stored at or below about -2 degrees C. for between six months and four years, or for an aqueous solution botulinum toxin containing pharmaceutical composition which has been stored at between about 2 degrees and about 8 degrees C. for from six months to four years, the botulinum toxin present in the reconstituted or aqueous solution pharmaceutical composition has greater than about 20% and up to about 100% of the toxicity that the biologically active botulinum toxin had prior to being incorporated into the pharmaceutical composition.

A pharmaceutical composition within the scope of the present invention includes a neurotoxin, and a polysaccharide. The polysaccharide stabilizes the neurotoxin. The pharmaceutical compositions disclosed herein can have a pH of between about 5 and 7.3 when reconstituted or upon injection. The average molecular weight of a disaccharide unit of the polysaccharide is preferably between about 345 D and about 2,000 D. In a more preferred embodiment, the average molecular weight of a disaccharide unit of the polysaccharide is between about 350 kD and about 1,000 kD and in a most preferred embodiment between about 375 D and about 700 D. Additionally, the polysaccharide can comprise at least about 70% amylopectin. Furthermore, the weight average molecular weight of the polysaccharide itself is between about 20 kD and about 2,500 kD.

Substantially all of the disaccharide units of the polysaccharide comprise ether linked glucopyranose molecules. An average of about 4 to about 10 of the hydroxyl groups present on each 10 of the glucopyranoses present in the polysaccharide are substituted, through an ether linkage, with a compound of the formula (CH₂)ₙ-OH, where n can be an integer from 1 to 4. More preferably, n is an integer between 1 and 3.

In a particularly preferred embodiment, an average of about 6 to about 9 of the hydroxyl groups present on each 10 of the glucopyranoses present in the polysaccharide are substituted, through an ether linkage, with a compound of the formula (CH₂)ₙ-OH, where n can be an integer from 1 to 4. And in a most preferred embodiment, an average of about 7 to about 8 of the hydroxyl groups present on each 10 of the glucopyranoses present in the polysaccharide are substituted, through an ether linkage, with a compound of the formula (CH₂)ₙ-OH, where n can be an integer from 1 to 4.

A detailed embodiment of the present invention can be a pharmaceutical composition suitable for injection into a human patient, which includes a botulinum toxin, and a polysaccharide. The polysaccharide can comprise a plurality of linked glucopyranose units, each glucopyranose unit having a plurality of hydroxyl groups, wherein an average of about 6 to about 9 of the hydroxyl groups present on each 10 of the glucopyranoses present in the polysaccharide are substituted, through an ether linkage, with a compound of the formula (CH₂)ₙ-OH, where n can be an integer from 1 to 4. The polysaccharide can be an ethyl ether substituted polysaccharide.

The pharmaceutical composition is suitable for administration to a human patent to achieve a therapeutic effect, and the neurotoxin can be one of the botulinum toxin serotypes A, B, C₁, D, E, F and G. In a preferred embodiment of the present invention, the pharmaceutical composition, comprises a botulinum toxin, and a hydroyethyl starch.

Another embodiment of the present invention can encompass a pharmaceutical composition which includes a botulinum toxin, a polysaccharide, and an amino acid or a polyamino acid.

Whether the pharmaceutical composition comprises, beside the neurotoxin active ingredient, only a polysaccharide stabilizer or both polysaccharide and amino acid stabilizers, the pharmaceutical composition retains its potency substantially unchanged for six month, one year, two year, three year and/or four year periods when stored at a temperature between about -1° C. and about -15° C. Additionally, the indicated pharmaceutical compositions can have a potency or % recovery of between about 20% and about 100% upon reconstitution. Alternately or in addition, the pharmaceutical composition can have a potency of between about 10 U/mg and about 30 U/mg upon reconstitution, such as a potency of about 20 U/mg upon reconstitution. Significantly, the pharmaceutical composition is devoid of any albumin. Thus, the pharmaceutical composition can be substantially free of any non-toxin complex proteins. Notably, the amino acid can be present in an amount of between about 0.5 mg and about 1.5 mg of amino acid per 100 units of botulinum toxin.

The polysaccharide can be a starch such as a hydroxyethyl starch, which, when the pharmaceutical composition comprises about 100 units of the botulinum toxin, there can be between about 500 µg and about 700 µg of the hydroxyethyl starch present. Preferably, the botulinum toxin is botulinum toxin type A, and the amino acid, when present, is selected from the group consisting of lysine, glycine, histidine and arginine.

A further detailed embodiment of the present invention can be a stable, high potency, non-pyrogenic, vacuum dried botulinum toxin type A pharmaceutical composition, comprising a botulinum toxin type A complex, a polysaccharide, and, an amino acid or a polyamino acid. The pharmaceutical composition can be albumin free, have a 1 year shelf life at -5 C with about 90% potency immediately upon reconstitution with saline or water and about 80% potency 72 hours after reconstitution and storage at 2 C. Additionally, the pharmaceutical composition can have a specific toxicity of at least about 18 U/mg upon reconstitution.

The present invention also encompasses a lyophilized or vacuum dried pharmaceutical composition consisting essentially of a high molecular weight polysaccharide and a botulinum toxin, wherein the botulinum toxin is stabilized by the high molecular weight polysaccharide. The high molecule weight polysaccharide can be selected from the group consisting of hydroxymethyl starch, hydroxyethyl starch, hydroxypropyl starch, hydroxybutyl starch, and hydroxypentyl starch and The botulinum toxin can be selected from the group consisting of botulinum toxin types A, B, C₁, D, E , F and G.

The polysaccharide can be present in the pharmaceutical composition in an amount of between about 1 X 10⁻⁹ moles of the polysaccharide per unit of a botulinum toxin to about 2 X 10⁻¹² moles of the polysaccharide per unit of the botulinum toxin.

The present invention also includes (a) a method for making a pharmaceutical composition, comprising the step of preparing a mixture of a botulinum toxin and a polysaccharide comprised of covalently linked repeating monomers, wherein the average monomer molecular weight is between about 350 D and about 1,000 D, and (b) a method for stabilizing a clostridial neurotoxin against denaturation or aggregation, the method comprising the step of contacting a clostridial neurotoxin with a stabilizing composition comprising a polysaccharide. The contacting step in this later method can comprise the step of adding to an aqueous solution or to a lyophilized or vacuum dried powder containing a clostridial neurotoxin an effective amount of the polysaccharide.

### DESCRIPTION

The present invention is based upon the discovery that a stable neurotoxin containing pharmaceutical composition can be formulated free of any animal derived protein or donor pool albumin by incorporating a polysaccharide and/or an amino acid into the pharmaceutical composition. In particular, the present invention is based upon the discovery that a stable botulinum toxin containing pharmaceutical composition suitable for administration to a human patient for therapeutic effect can be made by replacing the donor pool albumin present in known botulinum toxin containing pharmaceutical compositions with a high molecule weight polysaccharide derived from starch and/or with certain reactive amino acids.

Surprisingly, I have found that a suitable replacement for albumin is a compound which is neither another protein, nor a low molecular weight, non-protein compound. Thus, I have discovered that particular high molecular weight polysaccharides can function as neurotoxin stabilizers in a pharmaceutical composition. As set forth below an amino acid can also, or in the alternative, be added to the pharmaceutical composition to increase the stability and useful storage life of the pharmaceutical composition.

The polysaccharide used in the present invention can impart stability to a neurotoxin active ingredient, such as a botulinum toxin, present in the pharmaceutical composition by: (1) reducing adhesion (commonly referred to as "stickiness") of the botulinum toxin to surfaces, such as the surfaces of laboratory glassware, vessels, the vial in which the pharmaceutical composition is reconstituted and the inside surface of the syringe used to inject the pharmaceutical composition. Adhesion of the botulinum toxin to surfaces can led to loss of botulinum toxin and to denaturation of retained botulinum toxin, both of which reduce the toxicity of the botulinum toxin present in the pharmaceutical composition. (2) reducing the denaturation of the botulinum toxin and/or dissociation of the botulinum toxin from other non-toxin proteins present in the botulinum toxin complex, which denaturation and/or dissociation activities can occur because of the low dilution of the botulinum toxin present in the pharmaceutical composition (i.e. prior to lyophilization or vacuum drying) and in the reconstituted pharmaceutical composition. (3) reducing loss of botulinum toxin (i.e. due to denaturation or dissociation from non-toxin proteins in the complex) during the considerable pH and concentration which take place during preparation, processing and reconstitution of the pharmaceutical composition.

The three types of botulinum toxin stabilizations provided by the polysaccharide conserve and preserve the botulinum toxin with it native toxicity prior to injection of the pharmaceutical composition.

A preferred polysaccharide for use in the present composition comprises a plurality of glucose monomers (mol wt 180) with one or more substituents on a majority of the glucose monomers, so that the preferred polysaccharide has a molecular weight range of between about 20 kD and about 800 kD. Surprisingly, such a polysaccharide can stabilize a neurotoxin component present in a pharmaceutical composition. The present invention excludes from its scope disaccharide oligosaccharides with a weight average molecular weight of less than about 20 kD. The present invention also excludes from its scope cyclic polymers such as the cyclodextrins. The latter two classes of compounds are excluded from the scope of the present invention because the desired stabilization characteristics of the preferred polysaccharide while requiring a relatively high molecular compound (i.e. molecular weight in excess of 20 kD) do not require and indeed can make no use of the small lipophilic cavity characteristic of the cyclodextrins, because the cyclodextrin lipophilic cavity is much smaller in size than the size of the neurotoxins stabilized by the preferred polysaccharides of the present invention. Additionally, the cyclodextrins are low molecular weight compounds comprising only about 6 to 8 glucose monomers.

The present invention also encompasses a method for stabilizing pharmaceutical compositions which contain a clostridial toxin with a polysaccharide. The stabilizing effect is achieved by bringing a clostridial toxin in contact with the polysaccharide. Examples of suitable polysaccharides within the scope of my invention include certain starch derivatives. As noted, the polysaccharide exhibits a stabilizing effect on the clostridial toxin. Furthermore, the effect of the polysaccharide to stabilize a clostridial toxin can be enhanced by the addition of an amino acid.

Unexpectedly, I have discovered that 2-hydroxyethyl starch demonstrates a unique ability to stabilize the botulinum toxin present in a botulinum toxin containing pharmaceutical composition, thereby providing a pharmaceutical composition which is devoid of the potential for harboring a transmissible disease derived from human blood or blood fraction donor pools or animal derived proteins like gelatin.

Thus, I have discovered that the particular high molecular weight polysaccharide, hydroxyethyl starch, can stabilize the toxin during formulation, drying, storage and reconstitution. Preferably, to further stabilize the protein active ingredient, an amino acid is also included in the polysaccharide containing formulation.

The polysaccharide in the pharmaceutical composition is preferably admixed with the clostridial neurotoxin in an amount of about 1 µg of polysaccharide per unit of botulinum toxin to about 10 µg of polysaccharide per unit of botulinum toxin. More preferably the polysaccharide in the pharmaceutical composition is admixed with the clostridial neurotoxin in an amount of about 4 µg of polysaccharide per unit of botulinum toxin to about 8 µg of polysaccharide per unit of botulinum toxin. In a most preferred embodiment, where the polysaccharide is a hydroxyethyl starch, the hydroxyethyl starch in the pharmaceutical composition is preferably admixed with a botulinum toxin type A complex in an amount of about 5 µg of hydroxyethyl starch per unit of botulinum toxin to about 7 µg of hydroxyethyl starch per unit of botulinum toxin. Most preferably, the hydroxyethyl starch in the pharmaceutical composition is admixed with a botulinum toxin type A complex in an amount of about 6 µg of hydroxyethyl starch per unit of botulinum toxin. Since BOTOX® contains about 100 units of botulinum toxin type A complex per vial and the average molecular weight of hydroxyethyl starch is generally regarded as being between about 20 kD and about 2,500 kD, the most preferred concentration of hydroyethyl starch is between about 1 X 10⁻⁹ moles per unit of botulinum toxin (M/U) to about 2 X 10⁻¹² moles per unit of botulinum toxin. In another preferred embodiment, for a 100 U botulinum toxin type A complex pharmaceutical composition, about 600 µg of the hydroxyethyl starch and about 1 mg of an amino acid, such as lysine, glycine, histidine or arginine is included in the formulation. Thus, my invention encompasses us of both a polysaccharide and an amino acid or a polyamino acid to stabilize the neurotoxin active ingredient in the pharmaceutical composition.

Additionally, my invention also encompasses use of a suitable amino acid in a sufficient amount to stabilize the protein active ingredient in a pharmaceutical composition in the presence of the polysaccharide present in the formulation. Thus, I have surprisingly discovered that the inclusion of certain amino acids into a neurotoxin containing, pharmaceutical composition formulation can extend the useful shelf life of such a pharmaceutical composition. Thus, my invention encompasses a neurotoxin containing pharmaceutical composition which includes an amino acid and the use of such a pharmaceutical composition. Without wishing to be bound by theory, I can postulate that since a neurotoxin, such as a botulinum toxin, is susceptible to oxidation, due to the presence of disulfide linkages in the toxin complex, the inclusion of an oxidizable amino acid may act to reduce the probability that oxidizers, such as peroxides and free radicals, will react with the neurotoxin. Thus, the likelihood that the oxidizable neurotoxin disulfide linkage will be oxidized by an oxidizer, such as peroxides and free radicals, can be reduced upon inclusion of an amino acid which can act as an oxidative sink, that is as a scavenger for oxidizing compounds. A suitable amino acid is an amino acid which is subject to oxidation. Examples of preferred amino acids are methionine, cysteine, tryptophan and tyrosine. A particularly preferred amino acid is methionine.

A preferred embodiment of my invention can also include the use of two or more amino acids in combination with a polysaccharide to stabilize the protein active ingredient in a pharmaceutical composition. Thus, for a 100 U botulinum toxin type A containing pharmaceutical composition, about 0.5 mg of lysine and about 0.5 mg of glycine can be used, with between about 500 µg and about 700 µg of hetastarch.

Thus, as set forth above, my invention encompasses a protein containing, pharmaceutical composition which includes a polysaccharide. The polysaccharide acts to stabilize the protein active ingredient in the pharmaceutical composition. Additionally, my invention also includes protein containing, pharmaceutical composition which includes a polysaccharide and an amino acid. Surprisingly, I have discovered that the inclusion of certain amino acids into a neurotoxin containing, pharmaceutical composition formulation which includes a carbohydrate can extend the useful shelf life of such a pharmaceutical composition. Thus, my invention encompasses a neurotoxin containing pharmaceutical composition which includes both a polysaccharide and an amino acid and the use of such a pharmaceutical composition.

It is known that protein containing pharmaceutical compositions which also contain sugars, polysaccharides and/or carbohydrates (referred to hereafter as 'reactive compounds") are inherently unstable due to the fact that a protein and one of the three indicated reactive compounds can undergo the well-described Maillard reaction. Extensive, largely fruitless, research has been carried out to try and reduce the incidence or prevalence of this (for example) protein-polysaccharide Maillard reaction, by reduction of moisture or by the use of non-reducing sugars in the formulation. My discovery is based upon the observation that inclusion of a high concentration of a highly reactive amino acid encourages the Maillard reaction to take place between the stabilizing polysaccharide and the added amino acid. By providing an abundant amine source for the carbohydrate to react with, the probability of the protein drug (i.e. botulinum toxin active ingredient) becoming involved in the Maillard is reduced, thereby reducing this degradation pathway of the protein active ingredient and in this manner thereby stabilizing the protein active ingredient in the pharmaceutical composition.

Preferably, any compound containing a primary or secondary amine can be used for this purpose. Most preferred are amino acids, such as lysine, glycine, arginine. Polyamino acids, such as polylysine are also suitable. Cationic amino acids such as lysine may undergo ionic attraction, binding acidic proteins (e.g., botulinum toxins) and shield the active protein from contact with sugars. Polylysine, in addition to being larger and therefore more likely to act as a shield, provides the additional advantage of being antibacterial.

Another aspect of my invention is to pre-react the sugar and amino acid components to exhaust Maillard reaction potential before adding the active protein component (botulinum toxin) to the sugar and amino acid formulation ingredients, thereby substantially limiting the active protein's exposure to Maillard reactions.

Thus, my invention encompasses a pharmaceutical composition containing and the use of an amino acids and polyamino acids as Maillard reaction inhibitors in protein (i.e. botulinum toxin) drug formulations which contain polysaccharides.

The invention embodies formulations of active proteins (e.g., botulinum toxin) in combination with a stabilizing polysaccharide or combination of these and an amino acid such as lysine.

Significantly, I have discovered that hydroxyethyl starch does not undergo or undergoes a much attenuated rate or level of Maillard reactions with a protein, such as a botulinum toxin, when hydroxyethyl starch is compared to other polysaccharides or carbohydrates. Additionally, I have discovered that inclusion of an amino acid enhances the preservation effect of hydroxyethyl starch, possibly by acting as a competitive inhibitor, that is by competing with the toxin for Maillard reaction reactive sugars. For this purpose, amino acids such as lysine, glycine, arginine and histidine are preferred amino acids. Polyamino acids, such as polylysine, which exhibit the desired competitive inhibition behavior can also be used. Notably, the specified amino and poly amino acids can also exhibit antimicrobial properties, providing therefore the added benefit of reducing bacterial contamination in the pharmaceutical composition.

Reducing sugars, such as glucose and glucose polymers, undergo Maillard reaction with proteins. Even sugar alcohols like mannitol can react, albeit sometimes through contaminants or degradation products. Therefore a polysaccharide can stabilize the toxin for a period of time only to chemically react later, thereby causing reduced storage stability. It is obvious that the choice of polysaccharide is critical. I have discovered that the rate of hydroxyethyl starch participation in the Maillard reaction is very low. Additionally, I have found that hydroxyethyl cellulose, although structurally very similar to hydroxyethyl starch, is unsuitable to use as a stabilizer, since I have found that hydroxyethyl cellulose can rapidly react in a model system with lysine. This not only means that hydroxyethyl starch has an obvious advantage over other sugar (i.e. polysaccharide) stabilizers, but that even excipients similar to hydroxyethyl starch, such as hydroxyethyl cellulose, can be unsuitable to use as stabilizers of a protein active ingredient in a pharmaceutical formulation.

As noted, hydroxyethyl starch, can participate to at least some extent, in Maillard reactions. Thus, and as set forth above, a polysaccharide alone may not be sufficient to provide optimal stabilization of the toxin. Thus, I discovered the advantages of inclusion of an amino acid to act as a competitive inhibitor. Without wishing to be bound by theory, the hypothesis is that by providing another amine source, in high concentrations compared to the toxin, the probability of the Maillard reaction occurring with the toxin is reduced, thereby stabilizing the toxin. Any amino acid can be used however lysine being highly reactive and is a preferred amino acid.

My invention also encompasses addition of a zinc ion source to a botulinum toxin containing pharmaceutical formulation. Metals, especially divalent cations, are reported to greatly influence the success of a freeze-dried formulation due to various cryo-properties including the lattice structures of formed ices. Extraneous metals such as copper and iron species lend themselves to radical oxidations and are generally to be avoided. More specifically Botulinum Type-A toxin is dependent upon bound zinc for activity. Many of the proposed excipients or reaction products of the excipients will chelate metals. This could lead to formation of unstable ices and/or inactivated toxin. By supplying ample zinc in the formulation the presence of desirable divalent cations is assured, the likelihood of zinc loss by the toxin is reduced, and stability enhanced. This can be accomplished by the addition of ZnSO₄ to a botulinum toxin pharmaceutical composition.

### EXAMPLES

### Example 1 (comparative)

### Botulinum Toxin Pharmaceutical Composition

As previously set forth, botulinum toxin type A complex can be obtained from a culture of the Hall strain of Clostridium botulinum grown in a medium containing N-Z amine and yeast extract. The botulinum toxin type A complex is purified from the culture solution by a series of acid precipitations to a crystalline complex consisting of the active high molecular weight toxin protein and an associated hemagglutinin protein. The crystalline complex is then re-dissolved in a solution containing saline and albumin and sterile filtered (0.2 microns) prior to vacuum-drying. BOTOX® is then reconstituted with sterile, non-preserved saline prior to intramuscular injection. Each vial of BOTOX® contained about 100 units (U) of Clostridium botulinum toxin type A complex, 0.5 milligrams of human serum albumin and 0.9 milligrams of sodium chloride in a sterile, vacuum-dried form without a preservative. Alternately, the human serum albumin can be replaced by a recombinantly made albumin.

### Example 2

### Botulinum Toxin Pharmaceutical Composition Containing 2-Hydroxyethyl Starch

Botulinum toxin type A purified neurotoxin complex pharmaceutical formulations were prepared in the same manner set forth in Example 1 above, except that the 0.5 milligrams of albumin was replaced by either 500 µg or 600 µg of hetastarch. It was determined that full potency was maintained upon preparation of the hetastarch containing formulations. Thus, with both hetastarch containing formulations, the potency of the albumin-free, hetastarch containing composition, as measured at the time of reconstitution of the lyophilized, 100 U (± 20 U) botulinum toxin type A complex, was from 96 to 128 units. Three separate hetastarch, botulinum toxin type A complex pharmaceutical compositions had potency measurements, at the time of reconstitution, of, respectively, 105, 111 and 128 units. Potency was measured using the standard administration to mice toxin potency assay.

### Example 3

### Botulinum Toxin Pharmaceutical Composition Containing Glycine

Botulinum toxin type A purified neurotoxin complex pharmaceutical formulations was prepared in the same manner set forth in Example 1 above, except that the 0.5 milligrams of albumin was replaced by either 500 µg or 600 µg of hetastarch. In addition 1 mg of glycine was added to the formulation. A lyophilized, hetastarch plus glycine, albumin-free, 100 U botulinum toxin type A complex, pharmaceutical composition was then stored for seven months at -5° C. At the end of this seven month period the potency of this hetastarch plus glycine toxin formulation was determined, using the mouse administration assay, to be essentially unchanged (i.e. potency differed by less than 5% from the original potency).

### Example 4

### Botulinum Toxin Pharmaceutical Composition Containing Lysine

100 U botulinum toxin type A purified neurotoxin complex pharmaceutical formulations are prepared in the same manner set forth in Example 1 above, except that the 0.5 milligrams of albumin was replaced by 600 µg of hetastarch. In addition 1 mg of lysine is added to the formulation. A lyophilized, hetastarch plus lysine, albumin-free, 100 U botulinum toxin type A complex, pharmaceutical composition is then stored for one year month at -5° C. At the end of this one year period the potency of this hetastarch plus lysine, toxin formulation is determined, using the mouse administration assay, to be essentially unchanged (i.e. potency differs by less than 5% from the original potency).

### Example 5

### Botulinum Toxin Pharmaceutical Composition Containing Histidine

100 U botulinum toxin type A purified neurotoxin complex pharmaceutical formulations are prepared in the same manner set forth in Example 1 above, except that the 0.5 milligrams of albumin was replaced by 600 µg of hetastarch. In addition 1 mg of histidine is added to the formulation. A lyophilized, hetastarch plus histidine, albumin-free, 100 U botulinum toxin type A complex, pharmaceutical composition is then stored for one year month at -5° C. At the end of this one year period the potency of this hetastarch plus histidine, toxin formulation is determined, using the mouse administration assay, to be essentially unchanged (i.e. potency differs by less than 5% from the original potency).

### Example 6

### Botulinum Toxin Pharmaceutical Composition Containing Arginine

100 U botulinum toxin type A purified neurotoxin complex pharmaceutical formulations are prepared in the same manner set forth in Example 1 above, except that the 0.5 milligrams of albumin was replaced by 600 µg of hetastarch. In addition 1 mg of arginine is added to the formulation. A lyophilized, hetastarch plus arginine, albumin-free, 100 U botulinum toxin type A complex, pharmaceutical composition is then stored for one year month at -5° C. At the end of this one year period the potency of this hetastarch plus arginine, toxin formulation is determined, using the mouse administration assay, to be essentially unchanged (i.e. potency differs by less than 5% from the original potency).

### Example 7

### Use of a Botulinum Toxin Pharmaceutical Composition

A 48 year old male is diagnosed with a spastic muscle condition, such as cervical dystonia. Between about 10⁻³ U/kg and about 35 U/kg of a botulinum toxin type A pharmaceutical composition containing 600 µg of hetastarch and 1 mg of an amino acid, such as lysine, is injected intramuscularly into the patient. Within 1-7 days the symptoms of the spastic muscle condition are alleviated and alleviation of the symptoms persists for at least from about 2 months to about 6 months.

A pharmaceutical composition according to the invention disclosed herein has many advantages, including the following:
1. the pharmaceutical composition can be prepared free of any blood product, such as albumin and therefore free of any blood product infectious element such as a prion.
2. the pharmaceutical composition has stability and high % recovery of toxin potency comparable to or superior to that achieved with currently available pharmaceutical compositions.

Although the present invention has been described in detail with regard to certain preferred methods, other embodiments, versions, and modifications within the scope of the present invention are possible. For example, a wide variety of stabilizing polysaccharides and amino acids are within the scope of the present invention.

Accordingly, the scope of the following claims should not be limited to the descriptions of the preferred embodiments set forth above.

## Claims

1. A pharmaceutical composition suitable for injection into a human patient, comprising:
(a) a botulinum toxin, and;
(b) a polysaccharide comprising a plurality of linked glucopyranose units, each glucopyranose unit having a plurality of hydroxyl groups, wherein an average of about 4 to about 10 of the hydroxyl groups present on each 10 of the glucopyranoses present in the polysaccharide are substituted, through an ether linkage, with a compound of the formula (CH₂)ₙ-OH, where n can be an integer from 1 to 4.

2. The pharmaceutical composition of claim 1, wherein the average molecular weight of a disaccharide unit of the polysaccharide is between about 345 D and about 1,000 D.

3. The pharmaceutical composition of claim 1, wherein the polysaccharide is an ethyl ether substituted polysaccharide.

4. The pharmaceutical composition of claim 1, wherein the pharmaceutical composition is suitable for administration to a human patent to achieve a therapeutic effect.

5. The pharmaceutical composition of claim 1, wherein the botutinum toxin is selected from the group consisting of botulinum toxin types A, B, C₁, D, E, F and G.

6. A pharmaceutical composition according to claim 1, comprising a hydroxyethyl starch.

7. A pharmaceutical composition according to claim 1, additionally comprising (c) an amino acid or a polyamino acid.

8. The pharmaceutical composition of claim 7, wherein the pharmaceutical composition retains its potency substantially unchanged for one year when stored at a temperature between about -1° C. and about -15° C.

9. The pharmaceutical composition of claim 7, wherein the pharmaceutical composition retains its potency substantially unchanged for two years when stored at a temperature between about -1° C. and about -15° C.

10. The pharmaceutical composition of claim 7, wherein the pharmaceutical composition retains its potency substantially unchanged for three years when stored at a temperature between about -1° C. and about -15° C.

11. The pharmaceutical composition of claim 7, wherein the pharmaceutical composition retains its potency substantially unchanged for four years when stored at a temperature between about -1° C. and about -15° C.

12. The pharmaceutical composition of claim 7, wherein the pharmaceutical composition has a potency or % recovery of between about 20% and about 100% upon reconstitution.

13. The pharmaceutical composition of claim 7, wherein the pharmaceutical composition has a potency of between about 10 U/mg and about 30 U/mg upon reconstitution.

14. The pharmaceutical composition of claim 7, wherein the pharmaceutical composition has a potency of about 20 U/mg upon reconstitution.

15. The pharmaceutical composition of claim 7, wherein the pharmaceutical composition is devoid of any albumin.

16. The pharmaceutical composition of claim 7, wherein the pharmaceutical composition is substantially free of any non-toxin complex proteins.

17. The pharmaceutical composition of claim 7, wherein the polysaccharide is a starch.

18. The pharmaceutical composition of claim 7, wherein the Polysaccharide is a hydroxyethyl starch

19. The pharmaceutical composition of claim 18, wherein the pharmaceutical composition comprises about 100 units of the botulinum toxin, and between about 500 µg and about 700 µg of the hydroxyethyl starch.

20. The pharmaceutical composition of claim 19, further comprising between about 0.5 mg and about 1.5 mg of the amino acid

21. The pharmaceutical composition of claim 7, wherein the botulinum toxin is botulinum toxin type A.

22. The pharmaceutical composition of claim 7, wherein the amino acid is selected from the group consisting of lysine, glycine, histidine and arginine.

## Patentansprüche

1. Pharmazeutische Zusammensetzung, die zur Injektion bei einem menschlichen Patienten geeignet ist und die folgendes umfasst:
(a) ein Botulinumtoxin; und
(b) ein Polysaccharid, das eine Mehrzahl daran gebundener Glucopyranoseeinheiten umfasst, jede Glucopyranoseeinheit weist mehrere Hydroxylgruppen auf, worin durchschnittlich etwa 4-10 der Hydroxylgruppen, die auf jeweils 10 der Glucopyranosen in dem Polysaccharid vorhanden sind, über eine Etherbrücke mit einer Verbindung der Formel (CH₂)ₙ-OH, worin n eine ganze Zahl von 1-4 sein kann, substituiert sind.

2. Pharmazeutische Zusammensetzung gemäss Anspruch 1, worin das durchschnittliche Molekulargewicht einer Disaccharideinheit des Polysaccharids zwischen etwa 345 und 1.000 D liegt.

3. Pharmazeutische Zusammensetzung gemäss Anspruch 1, worin das Polysaccharid ein ethylethersubstituiertes Polysaccharid ist.

4. Pharmazeutische Zusammensetzung gemäss Anspruch 1, worin die pharmazeutische Zusammensetzung zur Verabreichung an einen menschlichen Patienten zur Erzielung einer therapeutischen Wirkung geeignet ist.

5. Pharmazeutische Zusammensetzung gemäss Anspruch 1, worin das Botulinumtoxin ausgewählt ist aus den Botulinumtoxintypen A, B, C₁, D, E, F und G.

6. Pharmazeutische Zusammensetzung gemäss Anspruch 1, die eine Hydroxyethylstärke umfasst.

7. Pharmazeutische Zusammensetzung gemäss Anspruch 1, die zusätzlich (c) eine Aminosäure oder eine Polyaminosäure umfasst.

8. Pharmazeutische Zusammensetzung gemäss Anspruch 7, worin die pharmazeutische Zusammensetzung ihre Stärke im wesentlichen unverändert beibehält, wenn sie für 1 Jahr bei einer Temperatur zwischen etwa -1 und -15°C gelagert wird.

9. Pharmazeutische Zusammensetzung gemäss Anspruch 7, worin die pharmazeutische Zusammensetzung ihre Stärke im wesentlichen unverändert beibehält, wenn sie für 2 Jahre bei einer Temperatur zwischen etwa -1 und -15°C gelagert wird.

10. Pharmazeutische Zusammensetzung gemäss Anspruch 7, worin die pharmazeutische Zusammensetzung ihre Stärke im wesentlichen unverändert beibehält, wenn sie für 3 Jahre bei einer Temperatur zwischen etwa -1 und -15°C gelagert wird.

11. Pharmazeutische Zusammensetzung gemäss Anspruch 7, worin die pharmazeutische Zusammensetzung ihre Stärke im wesentlichen unverändert beibehält, wenn sie für 4 Jahre bei einer Temperatur zwischen etwa -1 und -15°C gelagert wird.

12. Pharmazeutische Zusammensetzung gemäss Anspruch 7, worin die pharmazeutische Zusammensetzung nach Rekonstituierung eine Stärke oder prozentuale Wiederherstellung zwischen etwa 20 und 100 % aufweist.

13. Pharmazeutische Zusammensetzung gemäss Anspruch 7, worin die pharmazeutische Zusammensetzung nach Rekonstituierung eine Stärke zwischen etwa 10 und etwa 30 U/mg aufweist.

14. Pharmazeutische Zusammensetzung gemäss Anspruch 7, worin die pharmazeutische Zusammensetzung nach Rekonstituierung eine Stärke von etwa 20 U/mg aufweist.

15. Pharmazeutische Zusammensetzung gemäss Anspruch 7, worin die pharmazeutische Zusammensetzung von jeglichem Albumin frei ist.

16. Pharmazeutische Zusammensetzung gemäss Anspruch 7, worin die pharmazeutische Zusammensetzung von jeglichen Nichttoxin-Komplexproteinen weitgehend frei ist.

17. Pharmazeutische Zusammensetzung gemäss Anspruch 7, worin das Polysaccharid eine Stärke ist.

18. Pharmazeutische Zusammensetzung gemäss Anspruch 7, worin das Polysaccharid eine Hydroxyethylstärke ist.

19. Pharmazeutische Zusammensetzung gemäss Anspruch 18, worin die pharmazeutische Zusammensetzung etwa 100 Einheiten des Botulinumtoxins und zwischen etwa 500 und 700 µg Hydroxyethylstärke umfasst.

20. Pharmazeutische Zusammensetzung gemäss Anspruch 19, die ferner zwischen etwa 0,5 und 1,5 mg der Aminosäure umfasst.

21. Pharmazeutische Zusammensetzung gemäss Anspruch 7, worin das Botulinumtoxin Botulinumtoxin A ist.

22. Pharmazeutische Zusammensetzung gemäss Anspruch 7, worin die Aminosäure ausgewählt ist aus Lysin, Glycin, Histidin und Arginin.

## Revendications

1. Composition pharmaceutique appropriée pour une injection dans un patient humain, comprenant :
(a) une toxine botulinique et
(b) un polysaccharide comprenant une pluralité d'unités de glucopyranose liées, chaque unité de glucopyranose ayant une pluralité de groupes hydroxyles, où en moyenne d'environ 4 à environ 10 des groupes hydroxyles présents sur chaque 10 des glucopyranoses présents dans le polysaccharide sont substitués, via une liaison éther, par un composé de formule (CH₂)ₙ-OH, où n peut être un nombre entier de 1 à 4.

2. Composition pharmaceutique selon la revendication 1, dans laquelle le poids moléculaire moyen d'une unité disaccharide du polysaccharide est entre environ 345 D et environ 1 000 D.

3. Composition pharmaceutique selon la revendication 1, dans laquelle le polysaccharide est un polysaccharide à substitution éther éthylique.

4. Composition pharmaceutique selon la revendication 1, dans laquelle la composition pharmaceutique est appropriée pour une administration à un patient humain pour accomplir un effet thérapeutique.

5. Composition pharmaceutique selon la revendication 1, dans laquelle la toxine botulinique est sélectionnée dans le groupe consistant en les types A, B, C₁, D, E, F et G de toxine botulinique.

6. Composition pharmaceutique selon la revendication 1, comprenant un amidon hydroxyéthyle.

7. Composition pharmaceutique selon la revendication 1, comprenant de plus (c) un acide aminé ou un acide polyaminé.

8. Composition pharmaceutique selon la revendication 7, dans laquelle la composition pharmaceutique conserve sa puissance pratiquement inchangée pendant une année quand elle est stockée à une température entre environ -1°C et environ -15°C.

9. Composition pharmaceutique selon la revendication 7, dans laquelle la composition pharmaceutique conserve sa puissance pratiquement inchangée pendant deux années quand elle est stockée à une température entre environ -1°C et environ -15°C.

10. Composition pharmaceutique selon la revendication 7, dans laquelle la composition pharmaceutique conserve sa puissance pratiquement inchangée pendant trois années quand elle est stockée à une température entre environ -1 °C et environ -15°C.

11. Composition pharmaceutique selon la revendication 7, dans laquelle la composition pharmaceutique conserve sa puissance pratiquement inchangée pendant quatre années quand elle est stockée à une température entre environ -1°C et environ -15°C.

12. Composition pharmaceutique selon la revendication 7, dans laquelle la composition pharmaceutique a une puissance ou un % de récupération entre environ 20 % et environ 100 % après reconstitution.

13. Composition pharmaceutique selon la revendication 7, dans laquelle la composition pharmaceutique a une puissance entre environ 10 U/mg et environ 30 U/mg après reconstitution.

14. Composition pharmaceutique selon la revendication 7, dans laquelle la composition pharmaceutique a une puissance d'environ 20 U/mg après reconstitution.

15. Composition pharmaceutique selon la revendication 7, dans laquelle la composition pharmaceutique est exempte de toute albumine.

16. Composition pharmaceutique selon la revendication 7, dans laquelle la composition pharmaceutique est pratiquement exempte de toute protéine complexe non-toxine.

17. Composition pharmaceutique selon la revendication 7, dans laquelle le polysaccharide est un amidon.

18. Composition pharmaceutique selon la revendication 7, dans laquelle le polysaccharide est un amidon hydroxyéthylique.

19. Composition pharmaceutique selon la revendication 18, dans laquelle la composition pharmaceutique comprend environ 100 unités de la toxine botulinique et entre environ 500 µg et environ 700 µg de l'amidon hydroxyéthylique.

20. Composition pharmaceutique selon la revendication 19, comprenant en outre entre 0,5 mg et environ 1,5 mg de l'acide aminé.

21. Composition pharmaceutique selon la revendication 7, dans laquelle la toxine botulinique est une toxine botulinique de type A.

22. Composition pharmaceutique selon la revendication 7, dans laquelle l'acide aminé est sélectionné dans le groupe consistant en lysine, glycine, histidine et arginine.
